# EUROPEAN PATENT APPLICATION

(11) **EP 4 467 181 A1**
(43) Date of publication of application: **27.11.2024**
(21) Application number: 22919297.6
(22) Date of filing: 22.12.2022
(51) Int. Cl.: A61M 25/00, A61M 39/10, A61M 39/22, A61M 39/20, B01D 69/00, B01D 61/00

(54) **ADJUSTABLE FLOW URINARY CATHETER**

(30) Priority: 17.01.2022 ES 202230028
(71) Applicant: Rethink Medical SL, 35017 Las Palmas de Gran Canaria (ES)
(72) Inventor: LUQUE GONZÁLEZ, Manuel, 35017 LAS PALMAS DE GRAN CANARIA (Las Palmas) (ES); RUIZ CANALES, Jaime, 35017 LAS PALMAS DE GRAN CANARIA (Las Palmas) (ES); ENDRENYI, Szilvia, 35017 LAS PALMAS DE GRAN CANARIA (Las Palmas) (ES)
(74) Representative: Ponti & Partners, S.L.P
(86) International application number: PCT/ES2022/070831
(87) International publication number: WO 2023/135349

(57) **Abstract**

Urinary catheter (1) with slide valve (8) provided with a tube (3) and a moving element (2) with a narrowing and a relief (5) on the end face closest to the narrowing (13), where the tube (3) is provided with another relief (6) and both reliefs are at the same end of the slide valve (8) and close to each other. The valve also has curved surfaces (7) adapted to allow the fingers to make a firm, natural and ergonomic grip of the urinary catheter (1) and press the moving element (2).

## Description

### FIELD OF THE ART

The present invention belongs to the field of devices for removing agents from the body, and more specifically relates to a catheter or hollow probe.

The main object of the present invention is a urinary catheter that is inserted through the urethra or through a surgical orifice that connects it to the bladder. The urinary catheter incorporates at the opposite end, as part of its design, a valve that allows the user to regulate the discharge of urine to drain this fluid from the bladder, kidney or ureters, reducing the force required to actuate the valve integrated into the device.

### BACKGROUND OF THE INVENTION

As life expectancy has been increasing accompanied by medical advances, there are more factors that can cause a patient to suffer from acute urinary retention. It is estimated that at least 10% of people over the age of 65 suffer from benign prostatic hypertrophy that can cause acute urinary retention.

Urinary retention caused by trauma has not always had the same origin. In the past the reasons (accidental fall from a horse, injuries on the battlefield or of another origin) could be very different from those that produce this medical problem today, however, the result ended up being the same as today: an acute or chronic urinary retention. Once the bladder is full, it makes the patient's life incompatible since the pain that patients feel is extreme, and this is what probably led in different cultures to the use of utensils to relieve pain by draining the bladder.

The main reasons for requiring the insertion of a urinary catheter are the following:
- Allow urinary drainage in patients with neurological conditions that cause bladder dysfunction;
- Manage urinary incontinence in patients lacking cognitive function;
- Minimise skin degradation and pressure ulcers in paralysed, comatose or terminally ill patients;
- Irrigate the bladder;
- Administer chemotherapy;
- Assist in urological or other surgery on adjoining structures;
- Obtain accurate measurements of urinary discharge in critical or postoperative patients;
- Emptying the bladder during childbirth; and
- Conduct urodynamic studies (such as pressure measurements).

The first documented historical record of a bladder catheter is attributed to Galen and his *algalia* or *fistula aenea.* We do not know if this corresponds to an own invention or to modifications of an existing prototype, but we know that it was metallic, long and curved for men, and straight with half the length for women. These types of catheters persisted for more than ten centuries. In Muslim times it was made of silver, being lighter and finer. Throughout the Renaissance it remained with some small variations. In the seventeenth and eighteenth centuries there were no great variations except for the manufacture of some glass models in various countries.

Over time, advances in this device have initially focused on patient comfort, although since the invention of the Foley catheter, as we know it today, its concern has been more focused on the reduction of infections associated with bladder catheterisation, hence most of the innovations in this field that have reached the market are coatings or materials that reduce the proliferation of biofilms or bacteria.

Bladder drainage is usually performed by passing a Foley catheter, or an intermittent catheter, through the natural urethral duct (called transurethral catheterisation), or by creating an artificial path between the lower abdominal wall and the bladder (suprapubic catheterisation).

However, these bladder catheters have some disadvantages. At the time of insertion, urine leaks usually occur and sometimes end up producing biological contamination accidents that affect the professionals who insert them, since urine is often accompanied by blood. The bladder catheter usually requires connection to a urine collection bag which flows through the tube. This bag needs to be changed or emptied with a certain periodicity and as it is filled it involves an increase in the bag weight, thus limiting the patient's mobility. In addition, accidental disconnections of the collection bag may arise, as may occur with other accessories such as plugs or independent valves. On the other hand, the constant flow of urine into the collection bag produces biofilm inside the catheter tube, increasing the risk of infections, producing obstructions and increasing the frequency of visits to the emergency room or the doctor by the patient to solve these problems, which usually requires the insertion of a new catheter. Furthermore, the connection for long periods of the bladder catheter to the collection bag decreases the bladder reflex and produces inconveniences in the patient's life when it is removed.

Independent valves and other devices such as catheter caps are used as accessories to facilitate bladder emptying and dispensing with the collection bag. The valves require greater handling by the patient and professionals as they must be replaced periodically and may suffer accidental disconnections. In addition, they are not easy to use for all patients.

European patent no. EP2872194A1, by the same holder, discloses an improved urinary catheter, which has a flexible tube with an insertion section that allows it to be inserted through a urethra into the urinary bladder, the flexible tube having a connection section for connecting connection elements, wherein fluid can be drained from the urethra via the insertion section, and wherein a closable valve is arranged between the insertion section and the connection section, in such a way that the discharge of fluid from the connection section can be prevented while the valve is closed. The valve is configured as a slide valve, having three switching positions; closed (not allowing fluid to pass), open (allowing the passage of fluid and air) and a third position wherein only air is allowed to pass and not fluid.

After testing the prototypes and comments received from users on the previous catheter with no. EP2872194A1, a new catheter has been created with improvements to reduce the force required for its operation and reduce risks derived from the use, assembly and/or transport of the product.

### SUMMARY OF THE INVENTION

The device object of the present invention consists of a urinary catheter provided with a valve whose surfaces are curved so that the force necessary for its actuation is considerably reduced. The catheter is connected to the bladder by being inserted through the urethra or through a surgical orifice, having at the opposite end a connection cone to connect elements, such as collection bags, plugs and valves. In addition, the catheter comprises at the end opposite to the bladder connection zone a valve that allows regulating the discharge of fluid through the connection section, said valve being configured as a slide valve.

The slide valve allows regulating the passage of urine, without there being intermediate positions. The valve allows 3 possible positions that can be selected by users (professionals or patients):
- "Membrane position", 100% of the air flow can pass, preventing the entire passage of urine flow.
- "Open position", 100% of urine and air flow can pass.
- "Closed position", the entire passage of urine and air flow is prevented.

Depending on the type of user and the time of use:
- Health professionals, during the insertion process, can avoid contamination with urine, the sequence of use being as indicated below:
   ∘ With the slide valve of the device in the "membrane position", the device is inserted through the farthest part of said valve, until it accesses the bladder, then the urine begins to flow along the conduit of the device, pushing the air contained therein, so that when the air and the urine reach the location of the slide valve, at the end opposite to that located in the bladder, only the air can pass through the valve and exit to the outside, the urine being retained and occupying the entire volume and length of the catheter conduit.
   ∘ Then, the healthcare professional moves the slide valve to the "open position", allowing the controlled discharge of urine, which can be collected in the container prepared for this purpose for sampling, in addition to making sure that all the air has left the conduit. Whatever the motivation for opening the slide valve, it will always do so when and how desired, in short, in a controlled manner, avoiding unintentional splashes that can generate risk due to contamination.
   ∘ Finally, the healthcare professional moves the slide valve to the "closed position", preventing the discharge of urine, and leaving the device implanted in the patient, and waiting to be used as part of their daily life or as an auxiliary device during a hospital stay.
- Thanks to the controlled opening of the slide valve that allows regulating the passage of fluid, the patient, during his daily life, will be able to drain urine from the bladder naturally when he feels the need, or at intervals of time stipulated by the health professionals. This allows the urine to accumulate in the bladder and when it is released it can carry with it the biofilms that may have been generated in the tube, thus reducing the obstructions that frequently occur in bladder catheters. This implies that the patient does not need a collection bag, being able to connect it to the urinary catheter object of the present invention if desired. Not being dependent on the collection bag means a substantial improvement in the patient's quality of life, since said bag needs to be changed or emptied with a certain periodicity, and as it is filled it means an increase in its weight, thus limiting the patient's mobility. In addition, being able to dispense with the collection bag prevents accidental disconnections from it, which can also occur with other accessories such as plugs or independent valves. The patient's quality of life is also significantly improved in the medium/long term, since, by not producing a constant flow of urine into the collection bag, the generation of biofilm inside the tube is avoided, reducing the risk of infections, obstructions and reducing the frequency of visits to the emergency room or the doctor by the patient to solve these problems, which usually require the insertion of a new catheter. Finally, by not suffering the connection for long periods of the bladder catheter to the collection bag, the decrease in the bladder reflex is avoided, thus avoiding inconveniences in the life of the patient when the catheter is removed.

The urinary catheter object of the present invention comprises a main body made of silicone, or equivalent plastic material with similar qualities and properties, usually transparent, in order to be able to view the flow of urine therein.

The main body has a similar appearance to a Foley urinary catheter, so that, like this type of catheter, it comprises a tube that at one end has openings for the collection of urine and a balloon that, once inflated inside the bladder, prevents the catheter from coming out through the urethra. At the opposite end, the tube comprises two lumens, one for urine outlet and one for balloon inflation.

The slide valve of the invention is provided with curved surfaces so that its grip becomes easier and the force necessary for its activation decreases.

The main body, at the outlet end of the urine, incorporates a hollow or channel, preferably elliptical in section, which intersects with the conduit wherethrough the urine circulates, whose axis is preferably perpendicular to the urine flow conduit and which is open at both ends. In this hollow or channel is located, fitting integrally, without the possibility of movement, a tube of preferably elliptical section, hollow and made of rigid material, preferably of a plastic nature. The tube incorporates a lumen that always allows free passage to the fluid, since the regulation of the passage of the fluid is a function of the moving element of the slide valve. Inside this hollow tube runs the moving element of the slide valve that allows the passage of the fluid/urine to be regulated.

The moving element of the valve, which is ultimately the element that regulates the passage of fluid, consists of an elongated part, preferably elliptical in section, made of a rigid material, preferably plastic. It has a length with a lumen whose opening coincides with the urine flow channel when the moving element is moved by pushing through one of the ends. One of the two ends of the moving element is always visible and accessible by protruding from one of the ends of the rigid tube housed in the main body. Furthermore, the moving element has a narrowing in the longitudinal section such that, when the moving element is pushed and this narrowing coincides with the urine flow channel, it leaves free passage to the fluid, be it urine or air. The lumen has an attached element, a flexible membrane that only allows the passage of air but not urine, when both fluids run through the main body duct, coming from the bladder, looking for the exit to the outside.

The moving element comprises two chromatically differentiated parts, one part being preferably red and the other green. Each coloured part covers approximately half of the element's length, so that, depending on the position, we will have the following situations:
- "Open position", the mobile element at its green end is visible and fully accessible by one of the ends of the rigid tube that crosses the main body, while the red end is visible but not accessible, 100% of the urine and air flow being able to pass through in this situation.
- "Closed position", the mobile element at its red end is visible and fully accessible by one of the ends of the rigid tube that crosses the main body, while the green end is visible but not accessible, in this position the entire passage of urine and air being blocked.
- There is a third position that only occurs at the initial moment of use of the device, at the time of insertion into the patient by healthcare personnel. It is the so-called "membrane position", wherein 100% of the air flow can pass, preventing the entire passage of urine flow. With regard to the chromatic differences of, the moving element at its red end is visible and fully accessible through one end of the rigid tube passing through the main body, while the green end is visible but not accessible, as is the case with the "closed position", with the difference that the red length of the moving element that is visible is longer than in the case of the "closed position". During the insertion process, once the healthcare personnel have allowed the drainage of the air from the duct, the urine is retained without the possibility of an uncontrolled discharge, and occupying the entire volume and length of the catheter conduit.

At that time the healthcare professional will move the slide valve, passing first through the "open position" and finally, if he continues to push, the slide valve will move until it is in the "closed position". From the moment the closed position is reached, it is infeasible to return the slide valve to the "membrane position", leaving this position disabled during the useful life of the device, so that there is no possibility of visual confusion between the "closed position" and the "membrane position" since, during the useful life of the device, under normal conditions of use, only the "closed position" and the "open position" will be possible and accessible.

The tube of preferably elliptical section, hollow and made of rigid material, preferably plastic, located inside the hollow of the main body, has two ends always visible, and has in one of them a relief that visually and tactilely allows to identify said end of the element.

The moving slide valve, at the end that when visible corresponds to the "open position" and which is preferably green, has a relief that visually and tactilely identifies said end of the element.

Once the valve is inserted into the tube, the reliefs of the rigid tube and the slide valve must be next to each other on the same side.

The urinary catheter object of the present invention comprises a plug (4), rigid and preferably made of plastic material that fits at the end of the hollow, elliptical section tube, at the end opposite the end where both the moving element (2) and the tube (3) have reliefs. The plug (4) is fitted so that its entire length is inside the tube, except for a part that is outside the body of the tube, which prevents the part from advancing inside the tube (3). When the plug (4) is inside the tube, the slide valve is prevented from moving involuntarily, leaving the "membrane position" fixed in the urinary catheter object of the present invention, guaranteeing the healthcare professional that only air will pass through the duct and at no time the liquid content of the bladder.

### BRIEF DESCRIPTION OF THE DRAWINGS

To complement the present description, and to help to better understand the characteristics of the invention according to a preferred practical embodiment thereof, the said description is accompanied, as an integral part thereof, by a set of figures where the following has been represented in an illustrative and non-limiting manner:
Figure 1 shows a view of a possible embodiment of the device according to the present invention.
Figure 2 shows several views of a possible embodiment of the moving element of the slide valve.
Figure 3 shows several views of a possible embodiment of the tube of preferably elliptical, hollow section.
Figure 4 shows details of a possible embodiment of the reliefs in the tube of preferably elliptical, hollow section and in the moving element of the slide valve.
Figure 5 shows several views of a possible embodiment of the plug that allows to fix the "membrane position" of the device according to the present invention.
Figure 6 shows views and section of a possible embodiment of the device according to the present invention, the slide valve being in "membrane position", and the device being held so that the thumb is resting on its pad against the end of the moving element of the slide valve.
In a preferred embodiment, this end will be green in colour and will have at least one protuberance or relief that differentiates it from the opposite red end. The index and middle fingers hold the main body in a position such that, if pressed with the thumb, they would exert an equal force in the opposite direction, so that the moving element of the slide valve would move towards the "open position" and then to the "closed position".

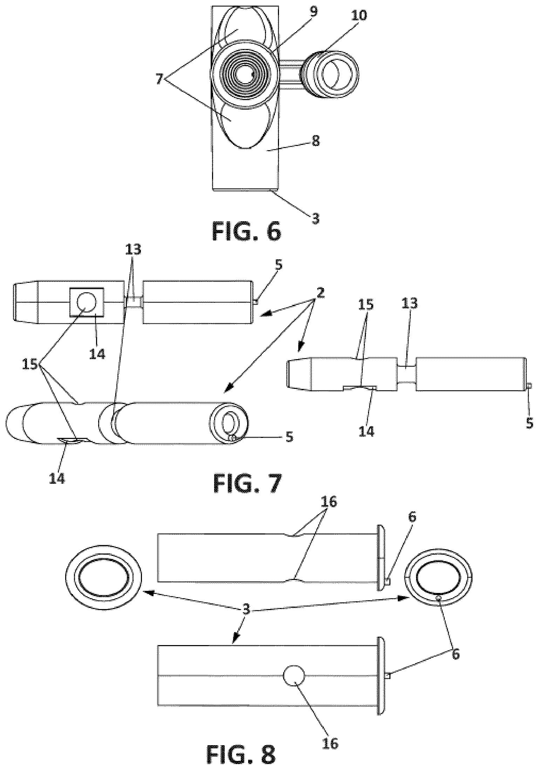

Figure 7 shows views and section of a possible embodiment of the device according to the present invention, the slide valve being in "closed position", and the device being held so that the thumb is resting on its pad against the end of the moving element of the slide valve. In a preferred embodiment, this end will be red, which differentiates it from the opposite green end. The index and middle fingers hold the main body in a position such that, if pressed with the thumb, they would exert an equal force in the opposite direction, so that the moving element of the slide valve would move towards the "open position".
Figure 8 shows views and section of a possible embodiment of the device according to the present invention, the slide valve being in "open position", and the device being held so that the thumb is resting on its pad against the end of the moving element of the slide valve. In a preferred embodiment, this end will be green, which differentiates it from the opposite red end. The index and middle fingers hold the main body in a position such that, if pressed with the thumb, they would exert an equal force in the opposite direction, so that the moving element of the slide valve would move towards the "closed position".

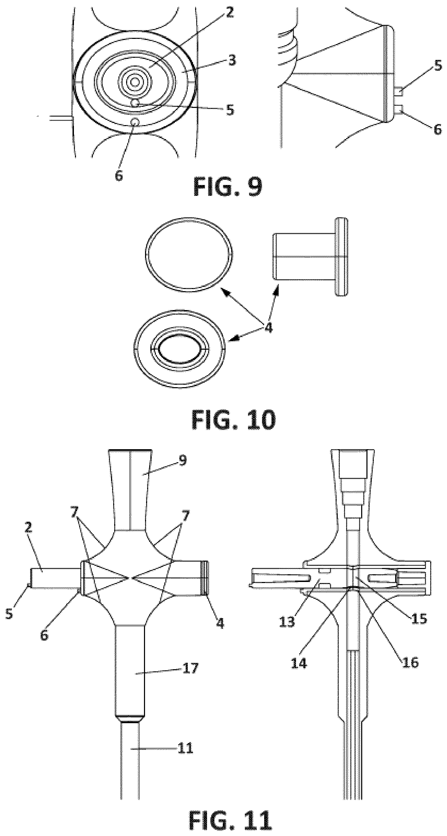

Figure 9 shows details of a possible embodiment of the protrusions or reliefs on the tube of preferably elliptical section, hollow and in the moving element of the slide valve.
Figure 10 shows several views of a possible embodiment of the plug allowing to fix the "membrane position" of the device in accordance with the present invention.
Figure 11 shows views and section of a possible embodiment of the device according to the present invention, being the sliding valve in "membrane position", with the plug in place, blocking the possibility of movement of the moving element of the sliding valve, so that the user has the guarantee that the valve is in "membrane position".

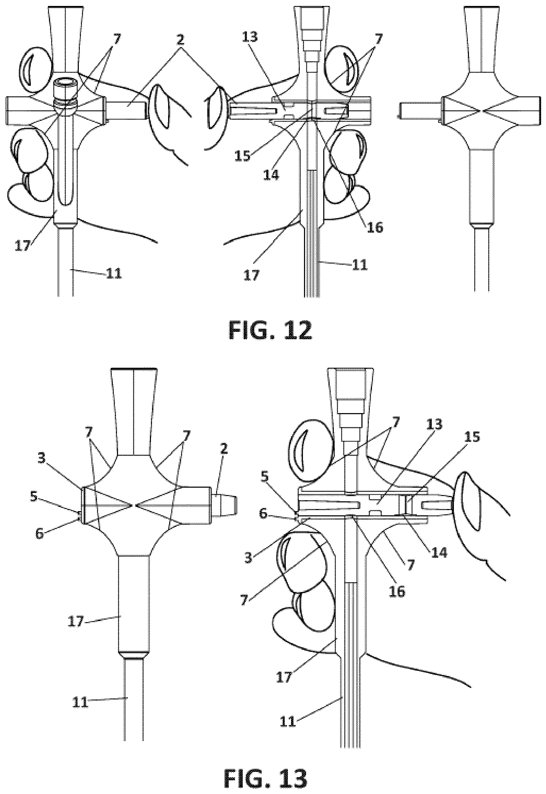

Figure 12 shows views and section of a possible embodiment of the device according to the present invention, the slide valve being in "membrane position", the device being held so that the thumb is resting on its pad against the end of the moving element of the slide valve which. In a preferred embodiment, this end will be green in colour and will have at least one protuberance or relief differentiating it from the opposite end, red in colour. The index and middle fingers hold the main body in such a position that, if pressed with the thumb, they would exert an equal and opposite force, so that the moving element of the slide valve would move to the "open position" and then to the "closed position".
Figure 13 shows views and section of a possible embodiment of the device according to the present invention, the slide valve being in "closed position" and the device being held so that the thumb is resting on its pad against the end of the moving element of the slide valve. In a preferred embodiment, this end will be red in colour differentiating it from the opposite green end. The index and middle fingers hold the main body in such a position that, in case of pressing with the thumb, they would exert an equal force and of opposite direction, so that the moving element of the sliding valve would move towards the "open position".

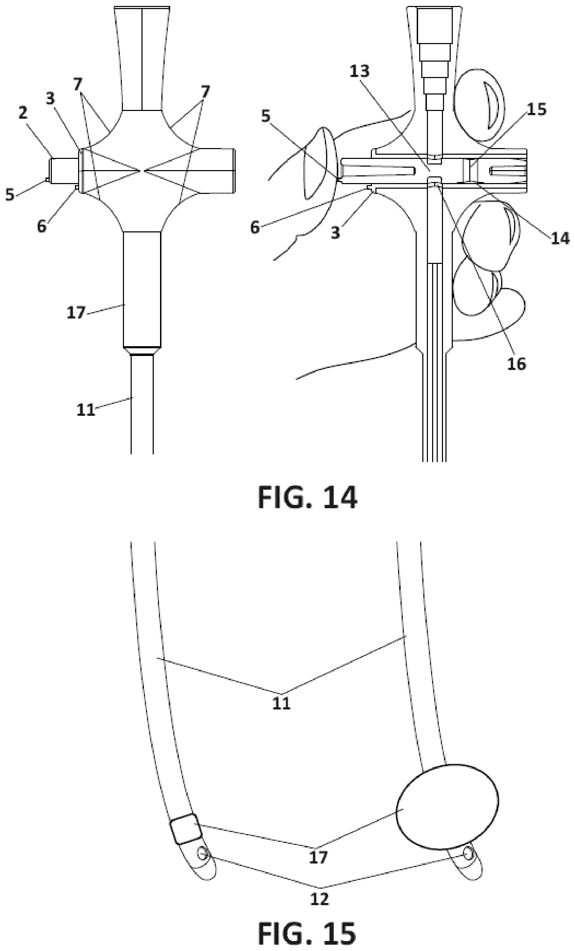

Figure 14 shows views and section of a possible embodiment of the device according to the present invention, the slide valve being in "open position" and the device being held so that the thumb is resting on its pad against the end of the movable element of the slide valve. In a preferred embodiment, this end will be green in colour distinguishing it from the opposite red end. The index and middle fingers hold the main body in such a position that, in case of pressing with the thumb, they would exert an equal and opposite force, so that the moving element of the slide valve would move towards the "closed position".
Figure 15 shows the end of the urinary catheter that connects to the bladder, on the left with the balloon that helps to retain the catheter inside the bladder deflated, on the left, and on the right the same balloon already inflated.

### PREFERRED EMBODIMENT OF THE INVENTION

In light of the aforementioned and described figures, and according to the adopted numbering, an example of a preferred embodiment of the invention can be observed therein, which comprises the parts and elements indicated and described below.

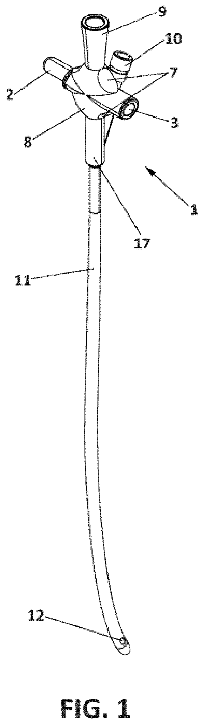

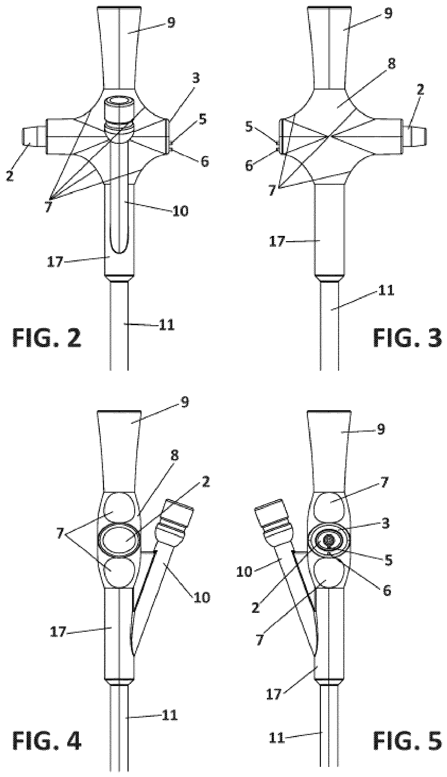

Thus, as can be seen in Figure 1, the urinary catheter (1) of the invention has an end (17) to connect to the bladder and a slide valve (8) consisting of the following elements:
- A moving element (2) consisting, as can be seen in Figure 2, of an elongated part, preferably elliptical in section, made of rigid material, preferably plastic, which has a narrowing (13) in the longitudinal section and a lumen (15). The lumen (15) will preferably be circular in section and will cross the entirety of the moving element (2) according to an axis parallel to the minor axis of the ellipse that preferably constitutes the section of the moving element (2). At one of the ends of the lumen (15) there is a surface on which an adhered element is housed, a membrane (14), which is a flexible sheet that allows only the passage of air but not of urine. The moving element (2) will have a relief (5) on the end face closest to the narrowing (13). The moving element (2) can be bicolour in a preferred embodiment, being of green colour, for example, the half that comprises from the narrowing (13) to the end that has the relief, while the rest will be of another different colour, red, for example.
- A tube (3) consisting, as can be seen in Figure 3, of an elongated element of preferably elliptical section, hollow and made of rigid material, preferably of a plastic nature. The tube, as seen in Figure 3, has a lumen (16) that always allows free passage to the fluid. In addition, it has at one of its ends, the one closest to the lumen (16), with a relief (5) that, visually and tactilely, allows identifying said end of the element.

In a preferred embodiment, the moving element (2) will be placed inside the tube (3), so that the relief (5) of the moving element (2) and the relief (6) of the tube (3) are at the same end of the slide valve (8), as can be seen in Figure 4. As can be seen in Figures 1, 2, 4, 6, 7 and 8, the moving element (2) always leaves one of its ends in view, while the rest of the volume remains inside the tube (3). The relief is shown in the figures as a point, but may have the shape of a thick curved line.

The slide valve is completed with surfaces (7) that, due to their curved shape, allow the fingers, for example index and heart, to make a firm, natural and ergonomic grip of the urinary catheter (1) in the area corresponding to the slide valve (8), in order to reduce the force exerted on the moving element (2) of the slide valve (8), by distributing the required force in three points, one in favour of the movement of the moving element and the other two in the opposite direction to its displacement. In this way, the movement of the moving element is favoured by means of a 3° lever. In a preferred embodiment, as can be seen in Figure 6, the urinary catheter (1) object of the present invention comprises a plug-shaped part (4) (Figure 5), rigid and preferably made of plastic material, which fits into the end of the tube (3) opposite to the one with the relief (6), so that when this plug is fitted to the bottom, the entire length is inside the tube, except for a protuberance that prevents its advancement inside the tube. The plug (4) prevents the slide valve from moving involuntarily, leaving the "membrane position" fixed in the urinary catheter (1) object of the present invention. This is the situation in which the healthcare professional finds the device at the time of its first use, just before insertion into the patient's urethra. In this way, the "membrane position" is guaranteed for the patient, thus being able to start the insertion procedure with all the guarantees for its correct development according to the steps described in the previous paragraphs.

In a preferred embodiment, as can be seen mainly in Figure 1, the urinary catheter (1), object of the present invention has an appearance very similar to that of a Foley type urinary catheter, and therefore has elements common to this type of devices, such as:
- A connection cone (9) at the distal end of the connection area with the urethra, as can be seen in Figures 1 to 6 and 11 to 13, which allows urine to be drained when the slide valve (8) is in the "open position" or, when necessary, to connect elements, such as collection bags, plugs and valves.
- A conduit (10) for inflation of the balloon (18), as seen in Figures 1, 2, 4, 5, 6. Said balloon is located at the end that is connected to the bladder, next to orifices for the collection of urine from the bladder and its subsequent conduction through the conduit (11) to the body (17), passing through the slide valve (8) where, in the case that said valve is in "open position", it exits outside the patient's body through the cone (9).

## Claims

1. Urinary catheter (1) comprising a main body (17) at the end whereof opposite the bladder connection area (12) is a slide valve (8), the slide valve being provided with a tube (3) consisting of an elongated element of elliptical section, hollow and made of rigid material and having a lumen (16) and a moving element (2) located inside the tube (3), where the moving element (2) comprises two chromatically differentiated parts and consists of an elongated part, of elliptical section, provided with a narrowing (13) in the longitudinal section and a lumen (15), where the lumen (15) is circular in section and crosses the entire moving element (2) according to an axis parallel to the minor axis of the ellipse that constitutes the section of said moving element (2), where at one of the ends of the lumen (15) there is a surface wherein a membrane (14) composed of a flexible sheet that only allows the passage of air but not urine is housed,
the urinary catheter being **characterised in that**:
- the moving element (2) is provided with a relief (5) on the end face closest to the narrowing (13), and the tube (3) is provided with another relief (6), and both reliefs are at the same end of the slide valve (8) and close to each other, and
- the slide valve (8) is provided with curved surfaces (7) adapted to allow the fingers to make a firm, natural and ergonomic grip of the urinary catheter (1) and press the moving element (2).

2. Urinary catheter (1) according to claim 1, comprising a part in the form of a rigid plug (4) made of plastic material, which fits into the end of the tube (3), at the end opposite to the end where the reliefs (5, 6) are located.
